# EUROPÄISCHE PATENTANMELDUNG

(11) **EP 1 616 863 A1**
(43) Veröffentlichungstag der Anmeldung: **18.01.2006**
(21) Anmeldenummer: 05013778.5
(22) Anmeldetag: 24.06.2005
(51) Int. Cl.: C07D 213/61, C07D 239/30

(54) **Verfahren zur Herstellung Kernfluorierter Aromaten**

(30) Priorität: 08.07.2004 DE 102004033525
(71) Anmelder: LANXESS Deutschland GmbH, 51369 Leverkusen (DE)
(72) Erfinder: Pleschke, Axel, Dr., 51069 Köln (DE); Marhold, Albrecht, Dr., 51373 Leverkusen (DE)

(57) **Zusammenfassung**

Die vorliegende Erfindung betrifft ein verbessertes Verfahren zur Herstellung kernfluorierter Aromaten der allgemeinen Formel (I) 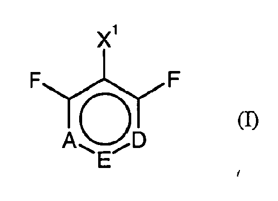 durch eine Halogenaustauschreaktion (Halex-Reaktion) mehrerer Halogensubstituenten in einer Stufe in Gegenwart eines Katalysators.

## Beschreibung

Die vorliegende Erfindung betrifft ein verbessertes Verfahren zur Herstellung kernfluorierter Aromaten durch eine Halogenaustauschreaktion (Halex-Reaktion) mehrerer Halogensubstituenten in einer Stufe in Gegenwart eines Katalysators.

Kernfluorierte Aromaten sind wichtige Zwischenprodukte zur Herstellung von biologisch aktiven Substanzen für pharmazeutische und agrochemische Anwendungen.

Es ist bekannt, Halex-Reaktionen in aprotischen, stark polaren Lösungsmitteln unter Einsatz von Metallfluoriden bei erhöhter Temperatur und in Gegenwart von Alkylammonium- oder Alkylphosphonium-Salzen (US-A 4 287 374), Pyridiniumsalzen (WO 87/04149), Kronenethern (DE-A 197 02 282) oder Tetraamidophosphoniumsalzen (WO 98/05610) durchzuführen. Nachteilig bei derartigen Reaktionen sind, insbesondere beim Einsatz schwach aktivierter Aromaten, die benötigten hohen Reaktionstemperaturen und langen Reaktionszeiten. Dies führt zu hohen Energieverbräuchen und niedrigen Raum-Zeit-Ausbeuten. Die hohen Reaktionstemperaturen führen häufig zur Bildung von unerwünschten Neben- und Zersetzungsprodukten. Außerdem benötigt man große Mengen teurer Lösungsmittel.

Insbesondere bereitet der Austausch mehrerer Halogensubstituenten schwach aktivierter Aromaten bisher allgemein Probleme.

So ist beispielsweise aus EP-A 1 266 904 bekannt, aromatische Verbindungen, die am Kern mit Halogen substituiert sind, mit einem Fluorid und bei Temperaturen zwischen 40 und 260°C und in Gegenwart eines speziellen Katalysators einer Halex-Reaktion zu unterziehen. Nachteilig bei dieser Reaktion ist jedoch, dass insbesondere ein Austausch von zwei und mehr Halogenatomen bei schwach aktivierten Aromaten, wie z.B. Tetrafluorbenzotrifluorid, eine mindestens zweistufige Reaktionsführung erforderlich macht, um die Menge an Fluorid und Lösungsmittel gering zu halten. Eine solche Zwei- bzw. Mehrstufigkeit der Reaktionsführung führt nicht nur aufgrund der Isolierung der Zwischenprodukte zu Ausbeuteverlusten, sondern auch zu höherem verfahrenstechnischem Aufwand. In 3,4-Dichlorbenzonitril wird sogar selektiv nur ein Chlorsubstituent gegen Fluor ausgetauscht.

Auch die Herstellung von 4,5,6-Trifluorpyrimidin mittels Halex-Reaktion ist in der Literatur bisher nur über die Zwischenstufe von 5-Chlor-4,6-dipyrimidin beschrieben (DE-A 196 02 095), welches seinerseits durch Halex-Reaktion aus 4,5,6-Trichlorpyrimidin hergestellt wird.

Der äußerst schwach aktivierte Aromat 1,3,5,-Triehlorbenzol kann lediglich bei Temperaturen von über 300°C durch Halex-Reaktion zu 1,3,5-Trifluorbenzol umgesetzt werden, wobei jedoch eine Realisierung im technischen Maßstab sehr aufwendig ist und bei diesen Temperaturen zu erheblichen Werkstoffproblemen, z.B. Spannungs-Risskorrosion bei Metallkesseln, führt.

Versuche, 1,3,5-Trifluorbenzol ausgehend von 1,3,5-Trichlorbenzol bei niedrigeren Temperaturen herzustellen führen entweder zu keinerlei Bildung des gewünschten Produkts (WO-A 02/092226) oder lediglich zu geringen Ausbeuten von maximal 14 % (WO-A 02/092608).

Auch bei der Umsetzung des schwach aktivierten 3,4,5-Trichlorbenzotrifluorid mit KF fällt neben 3-Chlor-4,5-difluorbenzotrifluorid als Hauptkomponente 3,4,5-Trifluorbenzotrifluorid lediglich als Nebenprodukt in einer Ausbeute von deutlich unter 10 % an.

Es bestand somit weiterhin Bedarf nach einer geeigneten Reaktionsführung, um zwei oder mehr Halogensubstituenten schwach aktivierter Aromaten in einer Halex-Reaktion gegen Fluorsubstituenten auszutauschen, die die vorangehend aufgeführten Nachteile bekannter Verfahren nicht aufweist und den entsprechenden kemfluorierten Aromaten in guter Ausbeute liefert.

Die Aufgabe, die der vorliegenden Erfindung zugrunde lag, war somit die Bereitstellung eines technisch weniger aufwändigen Verfahrens zur Herstellung kernfluorierter Aromaten durch Halogenaustauschreaktion (Halex-Reaktion) in guter Ausbeute.

Überraschend wurde nun gefunden, dass zwei- oder mehrfach kernfluorierte Aromaten in guter Ausbeute erhalten werden können, wenn zwei oder mehr Halogensubstituenten in entsprechend zwei- oder mehrfach kernhalogenierten schwach aktivierten Aromaten in Gegenwart von speziellen Katalysatoren in nur einem Reaktionsschritt ausgetauscht werden.

Gegenstand der vorliegenden Erfindung ist daher ein Verfahren zur Herstellung einer Verbindung der allgemeinen Formel (I), 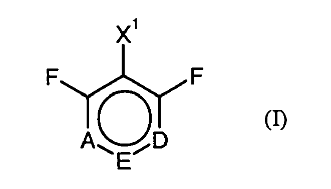
worin
- A: für N oder CH steht,
- D: für N oder CH steht,
- E: für N, CH, C-CF₃, C-CN oder CF steht und
- X¹: für H, CN oder F, bevorzugt für H oder F steht,
mit der Maßgabe, dass A, D und E nicht alle gleichzeitig dieselbe Bedeutung haben, bevorzugt nicht alle gleichzeitig für N oder nicht alle gleichzeitig für CH stehen,
durch Umsetzung einer Verbindung der allgemeinen Formel (II), 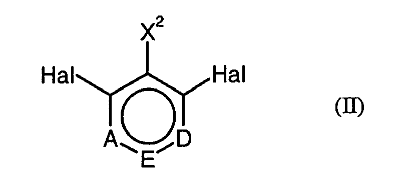
worin
- A: für N oder CH steht,
- D: für N oder CH steht,
- E: für N, CH, C-CF₃, C-CN oder C-Hal steht,
- X²: für H, Cl, Br, I oder CN, bevorzugt für H, Cl, Br oder CN, besonders bevorzugt für H oder Cl steht und
- Hal: für Cl, Br oder I, bevorzugt für Cl oder Br, besonders bevorzugt für Cl steht,
mit der Maßgabe, dass A, D und E nicht alle gleichzeitig dieselbe Bedeutung haben, bevorzugt nicht alle gleichzeitig für N oder nicht alle gleichzeitig für CH stehen,
mit einem Fluorid in Gegenwart mindestens einer Verbindung der allgemeinen Formel (III), 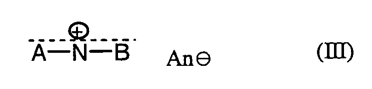 in der
- A: für einen Rest der Formeln (IV) oder (V) 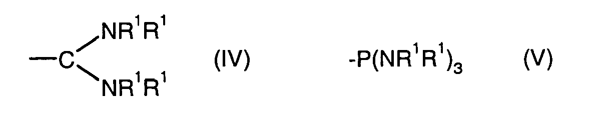
und
- B: unabhängig von A für einen Rest der Formeln (IV), (V), (VIa) oder (VIb) 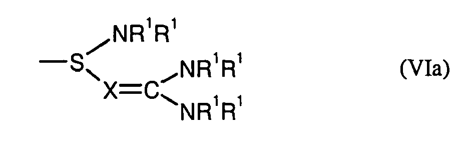
-S(NR¹R¹)₂ (VIb)
stehen,
wobei die einzelnen R¹ gleich oder verschieden sind und jeweils für geradkettiges oder verzweigtes C₁-C₁₀-Alkyl, geradkettiges oder verzweigtes C₂-C₁₀-Alkylen oder C₆-C₁₂-Aryl stehen,
wobei eine oder mehrere NR¹R¹-Gruppen auch für einen 3- bis 5-gliedrigen, gesättigten oder ungesättigten Ring stehen können, der aus einem N-Atom und sonst C-Atomen gebildet wird,
wobei die Formel (IV) und die 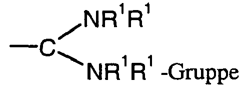 in Formel (VIa) auch für den Rest eines gesättigten oder ungesättigten 4- bis 8-gliedrigen Rings stehen kann, der zwei N-Atome und sonst C-Atome enthält,
- X: für N oder P steht und
- An^{⊖}: ein Äquivalent eines Anions bedeutet,

dadurch gekennzeichnet, dass die Umsetzung einstufig erfolgt.

Unter einer einstufigen Umsetzung ist im Rahmen der Erfindung zu verstehen, dass bei der Reaktionsführung keine Isolierung oder Aufarbeitung von teilfluorierten Zwischenprodukten erfolgt.

Bevorzugt eignet sich das erfindungsgemäße Verfahren zur Herstellung solcher Verbindungen der allgemeinen Formel (I) aus solchen Verbindungen der allgemeinen Formel (II), worin
A und D für N stehen und E für CH, C-CF₃ oder C-CN steht oder
A und D für CH stehen und E für N, C-CF₃ oder C-CN steht oder
A für N steht, D für CH steht und E für C-CF₃ steht oder
A und D für CH stehen und E in der allgemeinen Formel (I) für CF und in der allgemeinen Formel (II) für C-Hal steht, wobei Hal die oben für die allgemeine Formel (II) genannte Bedeutung hat.

Besonders bevorzugt eignet sich das erfindungsgemäße Verfahren zur Herstellung von 3,5-Difluorpyridin, 4,5,6-Trifluorpyrimidin, 1,3,5-Trifluorbenzol oder 3,4,5-Trifluorbenzotrifluorid, insbesondere zur Herstellung von 4,5,6-Trifluorpyrimidin, 1,3,5-Trifluorbenzol oder 3,4,5-Trifluorbenzotrifluorid. Als Verbindungen der allgemeinen Formel (II) werden hierzu besonders bevorzugt 3,5-Dichlorpyridin, 4,5,6-Trichlorpyrimidin, 1,3,5-Trichlorbenzol oder 3,4,5-Trichlorbenzotrifluorid, insbesondere 4,5,6-Trichlorpyrimidin, 1,3,5-Trichlorbenzol oder 3,4,5-Trichlorbenzotrifluorid eingesetzt.

Bei den gemäß erfindungsgemäßem Verfahren eingesetzten Verbindungen der allgemeinen Formel (II) handelt es sich um schwach aktivierte Aromaten, d.h. solche Aromaten, die eine mäßig elektronenziehende Gruppe in meta-(m-)Stellung zu mindestens einem, bevorzugt zu mindestens zwei der auszutauschenden Halogensubstituenten aufweisen. Unter mäßig elektronenziehenden Gruppen sind im Rahmen der Erfindung, unter der Berücksichtigung der vorzugsweise gegebenen meta-Stellung bezüglich mindestens einem, bevorzugt zu mindestens zwei der auszutauschenden Halogene, insbesondere N-Atome im aromatischen Ring sowie Halogen-, CF₃- oder CN-Substituenten an einem Ring-C-Atom zu verstehen, wobei Halogen sowohl für Cl, Br oder I als auch für bereits ausgetauschtes F stehen kann.

Vorzugsweise sind die beiden an das gleiche Stickstoffatom gebundenen R¹-Reste in den allgemeinen Formeln (IV), (V), (VI-a) und (VI-b) gleich.

Vorzugsweise stehen die Reste R¹ für Methyl, Ethyl, n-Propyl, i-Propyl, n-Butyl, sek-Butyl oder tert-Butyl, bevorzugt für Methyl, Ethyl, n-Propyl oder n-Butyl, oder eine NR¹R¹-Gruppe für einen 5- bis 7-gliedrigen gesättigten oder ungesättigten Ring, der aus einem Stickstoffatom und sonst C-Atomen gebildet wird
oder die Formel (IV) oder die 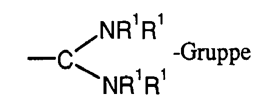 in Formel (VIa) für einen gesättigten 5- bis 7-gliedrigen Ring, der 2 Stickstoffatome und sonst Kohlenstoffatome enthält,
- X: für Stickstoff und
- An^{⊖}: für Chlorid, Bromid, (CH₃)₃SiF₂^{⊖}, HF₂^{⊖}, H₂F₂^{⊖}, Tetrafluoroborat, Hexafluorophosphat, Carbonat oder Sulfat.

Besonders bevorzugte Verbindungen der allgemeinen Formel (III) sind Verbindungen der Formeln (III-1) bis (III-6)

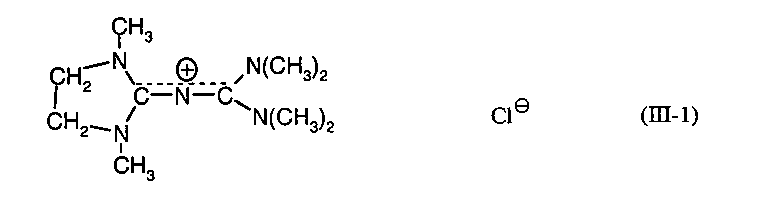

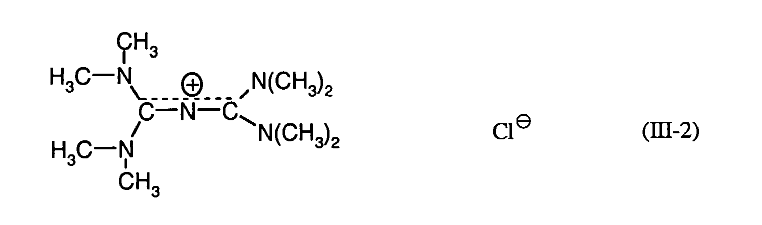

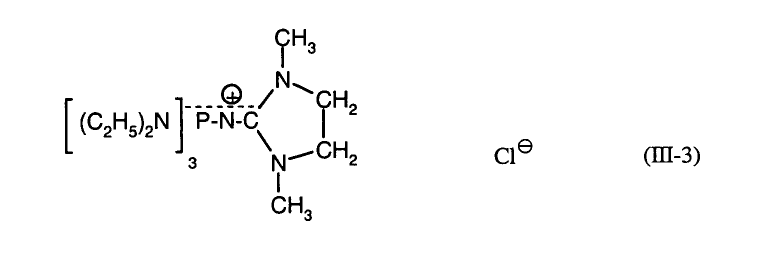

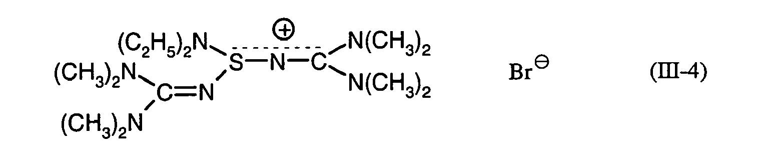

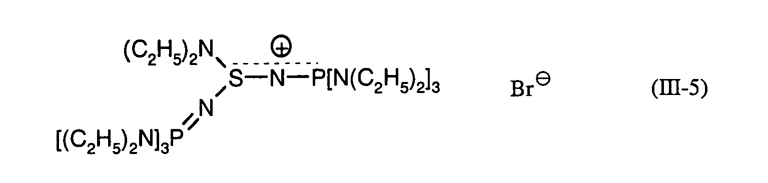

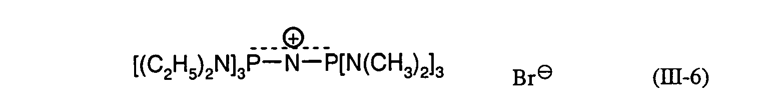

Als Fluoride zum Austausch von Halogen gegen Fluor kommen beispielsweise Alkali-, Erdalkali- und Ammoniumfluoride in Frage. Bevorzugt sind Kaliumfluorid, Natriumfluorid, Calciumfluorid und Ammoniumfluorid sowie deren Gemische untereinander sowie deren Gemische mit Lithium-, Rubidium- und/oder Cäsiumfluorid.

Bezogen auf 1 Mol gegen Fluor auszutauschendes Halogen, das an den Kern einer aromatischen Verbindung gebunden ist, kann man beispielsweise 0,001 bis 0,5 Mole, vorzugsweise 0,01 bis 0,1 Mole einer oder mehrerer Verbindungen der Formel (III) und beispielsweise 0,8 bis 2 Äquivalente, vorzugsweise 1,1 bis 1,5 Äquivalente eines oder mehrerer Fluoride einsetzen.

Die Verbindung(en) der allgemeinen Formel (III) können in isolierter Form oder in Form von Lösungen eingesetzt werden. Als geeignete Lösungsmittel kommen beispielsweise dipolar aprotische und/oder unpolar aprotische Lösungsmittel in Frage.

Das erfindungsgemäße Verfahren wird vorzugsweise bei Temperaturen im Bereich 40 bis 260°C, besonders bevorzugt bei 70 bis 240°C durchgeführt. Ganz besonders bevorzugt sind 160 bis 220°C.

Man kann das erfindungsgemäße Verfahren in Anwesenheit oder in Abwesenheit von Lösungsmitteln durchführen. Bevorzugt wird das erfindungsgemäße Verfahren in Anwesenheit von wenigstens einem Lösungsmittel durchgeführt. Als geeignete Lösungsmittel kommen beispielsweise dipolar aprotische und/oder unpolar aprotische Lösungsmittel in Frage. Geeignete dipolar aprotische Lösungsmittel sind beispielsweise Dimethylsulfoxid, Sulfolan, Dimethylformamid, Dimethylacetamid, 1,3-Dimethylimidazolin-2-on, N-Methylpyrrolidon, Acetonitril und Benzonitril. Geeignete unpolare aprotische Lösungsmittel sind beispielsweise Benzol, Toluol, Chlorbenzol, Dichlorbenzole, Chlortoluole und Chloralkane wie Dichlormethan. Die vorangehend aufgeführten Lösungsmittel eignen sich ebenfalls für die Lösungen der Verbindung(en) der allgemeinen Formel (III).

Unpolar aprotische und dipolar aprotische Lösungsmittel kann man in beliebigen Mengen einsetzen, beispielsweise in Mengen von 0,1 bis 500 Gew.-%, vorzugsweise in Mengen von 0,2 bis 300 Gew.-%, jeweils bezogen auf die eingesetzte aromatische Verbindung, die mit gegen Fluor austauschbarem Halogen substituiert ist.

Man kann auch Gemische von Lösungsmitteln einsetzen, wobei es bevorzugt ist solche Lösungsmittelgemische einzusetzen, die 50 Gew.-% oder mehr dipolar aprotische Lösungsmittel enthalten.

Man kann das erfindungsgemäße Verfahren weiterhin in Anwesenheit oder in Abwesenheit von Radikalfängern durchführen. Als geeignete Radikalfänger kommen beispielsweise aromatische Nitroverbindungen, bevorzugt Nitrobenzol, 3-Nitrodimethylbenzamid oder 1,3-Dinitrobenzol, besonders bevorzugt Nitrobenzol oder 3-Nitrodimethylbenzamid in Frage. Die Radikalfänger können in einer-Menge-von bis zu 10 Mol-% bezogen auf die Stoffmenge der Verbindung der allgemeinen Formel (II) eingesetzt werden. Durch den Einsatz von Radikalfängern kann die Bildung von Nebenprodukten durch Dehalogenierung deutlich vermindert werden.

Die Reaktionszeit beim erfindungsgemäßen Verfahren kann beispielsweise im Bereich von 2 bis 48 Stunden liegen.

Das erfindungsgemäße Verfahren kann bei vermindertem, normalem oder erhöhtem Druck durchgeführt werden. Vorzugsweise arbeitet man bei Normaldruck oder erhöhtem Druck, z.B. bei 1 bis 16 bar, besonders bevorzugt bei 2 bis 10 bar.

Grundsätzlich können die Verbindungen der Formel (I) in Anwesenheit oder Abwesenheit von Luftsauerstoff gehandhabt werden. Es ist jedoch bevorzugt, die Verbindungen der Formel (I) unter Schutzgas zu handhaben und das erfindungsgemäße Verfahren unter Schutzgas durchzuführen. Geeignete Schutzgase sind beispielsweise Stickstoff und Argon.

Das erfindungsgemäße Verfahren lässt sich diskontinuierlich oder kontinuierlich durchführen.

Zur Aufarbeitung des nach Durchführung des erfindungsgemäßen Verfahren vorliegenden Reaktionsgemisches kann man beispielsweise so verfahren, dass man das Reaktionsgemisch nach Abkühlung mit Wasser mischt, die sich bildende organische Phase abtrennt und die abgetrennte organische Phase bei reduziertem Druck fraktioniert destilliert. Man kann das nach Durchführung des erfindungsgemäßen Verfahrens vorliegende Reaktionsgemisch auch direkt einer Destillation unterwerfen. Ferner kann man dem Reaktionsgemisch ein Lösungsmittel zusetzen, feste Bestandteile durch Filtration abtrennen und das Filtrat bei reduziertem Druck destillieren. Des Weiteren kann das Produkt der allgemeinen Formel (I) auch mittels Destillation unter erhöhtem Druck (Druckdestillation) aus dem Reaktionsgemisch entfernt werden. Es sind auch andere Aufarbeitungsmöglichkeiten anwendbar.

Die Herstellung der Katalysatoren der allgemeinen Formel (III) ist bekannt und beispielsweise in Synthesis 1979, 215-216, Angewandte Chemie 104, 864, 1992 oder EP-A 1 266 904 beschrieben. In einigen Fällen ist beobachtet worden, dass bei der Herstellung von Verbindungen der Formel (III) Gemische von zwei oder mehr Einzelverbindungen anfallen, die der Formel (III) entsprechen. Auch solche Stoffgemische sind als Katalysatoren für das erfindungsgemäße Verfahren geeignet.

Das erfindungsgemäße Verfahren zur Herstellung von zwei- oder mehrfach kernfluorierten Aromaten erfolgt im Vergleich zu bekannten Verfahren einstufig und erfordert somit gegenüber dem Stand der Technik geringeren verfahrenstechnischen Aufwand bezüglich Materialien, Energie und Kesselbelegung. Zudem können die Zielprodukte gegenüber dem Stand der Technik mit mindestens vergleichbarer zumeist höherer Ausbeute erhalten werden. Bei dem erfindungsgemäßen Verfahren handelt es sich daher um ein gegenüber dem Stand der Technik deutlich verbessertes Verfahren.

Die folgenden Beispiele dienen der beispielhaften Erläuterung der Erfindung und sind nicht als Beschränkung aufzufassen.

### Beispiele

### Herstellung der Verbindung der Formel (III-1) (CNC-Katalysator)

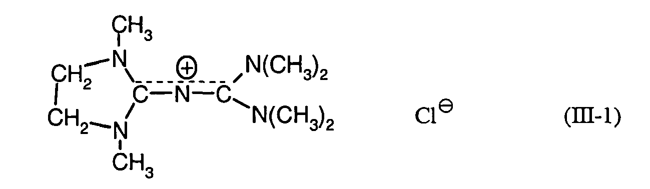

### (N,N-Dimethylimidazolidino)-tetramethylguanidinium-chlorid

Es wurden 600 ml Toluol vorgelegt und 360 g Phosgen innerhalb von 3,5 h bei Raumtemperatur (23°C) zugegeben. Anschließend werden 344 g (3.00 mol) 1,3-Dimethylimidazolidinon in 450 ml Toluol innerhalb 1.5 h zugegeben, wobei die Temperatur bei 40°C gehalten wird. Nach Ende der Gasentwicklung wird überschüssiges Phosgen durch Ausblasen mit Stickstoff entfernt. Die Suspension wird unter Inertgas filtiert und 438 g (2.56 mol) (N,N-Dimethylimidazolidino)-chlorid werden als farbloser Feststoff erhalten. Ausbeute: 85 %. Smp.: 156-158 °C.

600 ml Dichlormethan und 400g (2.34 mol) (N,N-Dimethylimidazolidino)-chlorid werden vorgelegt. Anschließend werden 552 g Tetramethylguanidin (2 eq., 4.8 mol) innerhalb 2 Stunden unter Kühlung zugegeben. Anschließend wird das Lösungsmittel entfernt und zu dem festen Rückstand werden 600 ml Methanol gegeben. Danach werden 432 g (2.4 mol) 30%ige Natriummethylat-Lsg (in Methanol) unter Kühlung zu der Suspension gegeben und eine Stunde bei Raumtemperatur gerührt. Die Lösungsmittel werden entfernt und anschließend werden 200 ml Dichlormethan zu dem Rückstand gegeben. Der Niederschlag wird abfiltriert und verworfen. Das Filtrat wird bis zur Trockne eingeengt. Man isoliert 449 g (1.80 mol) (N,N-Dimethylimidazolidino)-tetramethylguanidinium-chlorid (CNC-Katalysator der Formel (III-1)). Ausbeute: 94 %; Smp: 145-147°C

### Beispiel 1: Herstellung von 3,5-Difluorpyridin

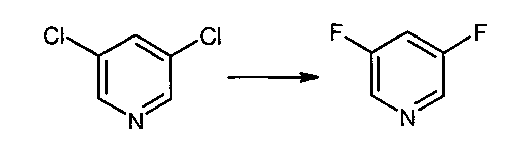

1000 g Dichlorpyridin und 1580 g trockenes Kaliumfluorid wurden in 1700 ml Sulfolan in einem Autoklaven vorgelegt. Anschließend wurden 84 g CNC-Katalysator (Verbindung (III-1)) zugesetzt, 3 bar Stickstoff aufgedrückt und für 48 h unter Rühren auf 205°C erwärmt. Während der Reaktion entstand ein maximaler Gesamtdruck von 12,4 bar. Anschließend wurde auf 10°C abgekühlt und das Produkt bei Normaldruck abdestilliert. Nach Redestillation wurden 473 g Difluorpyridin (60% der Theorie) als farblose Flüssigkeit erhalten.

### Beispiel 2: Herstellung von 4,5,6-Trifluorpyrimidin

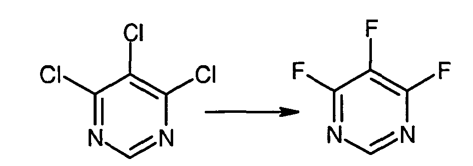

7160 ml Sulfolan und 3876 g KF wurden in einem Autoklaven vorgelegt und 1 h bei 150°C gerührt. Anschließend wurden zum Trocknen 700 ml Sulfolan im Vakuum abdestilliert. Daraufhin wurde auf 90°C abgekühlt, mit Stickstoff belüftet und eine auf 45°C gewärmte Lösung aus 3122 g 4,5,6-Trichlorpyrimidin und 2386 ml trockenem Sulfolan zugepumpt. Danach wurden 166 g CNC-Katalysator und 20,5 g Nitrobenzol schnell zugegeben und der Behälter verschlossen. Es wurde für 5 h unter Rühren auf 200°C und anschließend für 11 h auf 220°C erwärmt. Während der Reaktion entstand ein maximaler Gesamtdruck von 6,5 bar. Der Ansatz wurde unter Rühren auf 40°C abgekühlt und langsam in eine eisgekühlte Vorlage entspannt. Die Innentemperatur wurde langsam bis 150°C gesteigert und das Produkt zunächst drucklos , im weiteren Verlauf im Vakuum abdestilliert. Nach Redestillation des Rohprodukts wurden 1540 g 4,5,6-Trifluorpyrimidin (66 % d. Th.) als farblose Flüssigkeit erhalten.

### Beispiel 3: Herstellungsvorschrift von 3,4,5-Trifluorbenzotrifluorid

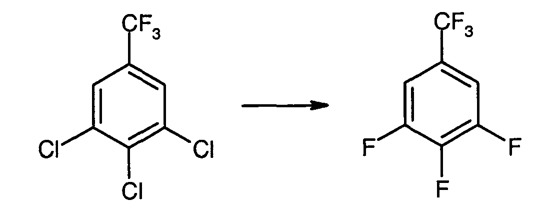

860 ml trockenes Sulfolan und 524 g trockenes KF wurden in einem Autoklaven unter Feuchtigkeitsausschluss vorgelegt und 500 g 3,4,5-Trichlorbenzotrifluorid, 5 g Nitrobenzol und 25 g CNC-Katalysator zugegeben. Der Behälter wurde verschlossen und der Ansatz anschließend für 5 h auf 200°C und dann weitere 12 h auf 220°C erwärmt. Während der Reaktion entstand ein maximaler Gesamtdruck von 9 bar. Danach wurde auf 20°C abgekühlt und langsam in eine gekühlte Vorlage entspannt. Das Produkt wurde bei Normaldruck abdestilliert. Nach Redestillation des Rohprodukts wurden 300 g 3,4,5-Trifluorbenzotrifluorid (75% d. Th.) als farblose Flüssigkeit erhalten.

### Beispiel 4: Herstellungsvorschrift von 1,3,5-Trifluorbenzol

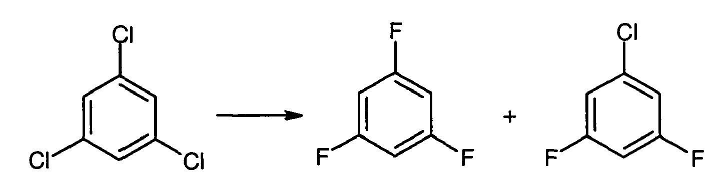

500 g 1,3,5-Trichlorbenzol, 1180 ml Sulfolan, 10.7 g 3-Nitrodimethylbenzamid und 640 g trockenes KF wurden in einem Autoklaven vorgelegt, anschließend 48 g CNC-Katalysator zugegeben und der Autoklav verschlossen. Es wurde für 48 h auf 220°C erhitzt. Während der Reaktion entstand ein maximaler Gesamtdruck von 8 bar. Anschließend wurde auf 20°C abgekühlt. Das Produkt wurde zunächst bei Normaldruck, im weiteren Verlauf im Vakuum abdestilliert. Es wurden 310 g einer farblosen Flüssigkeit mit einem Anteil von 87 Gew.-% 1,3,5-Trifluorbenzol (74 % d.Th.) und 8.8 Gew.-% Difluorchlorbenzol (6.7 % d.Th.) erhalten. 1,3,5-Trifluorbenzol und Difluorchlorbenzol sind auf bekannte Weise destillativ zu trennen.

## Patentansprüche

1. Verfahren zur Herstellung einer Verbindung der allgemeinen Formel (I), 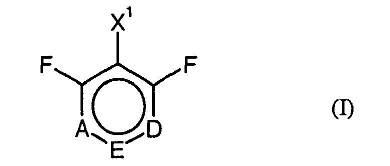 worin
A für N oder CH steht,
D für N oder CH steht,
E für N, CH, C-CF₃, C-CN oder CF steht und
X¹ für H, CN oder F steht,
mit der Maßgabe, dass A, D und E nicht alle gleichzeitig dieselbe Bedeutung haben,
durch Umsetzung einer Verbindung der allgemeinen Formel (II), 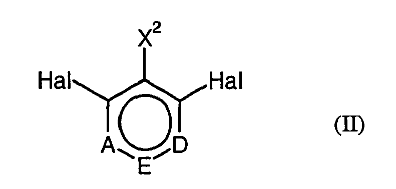 worin
A für N oder CH steht,
D für N oder CH steht,
E für N, CH, C-CF₃, C-CN oder C-Hal steht,
X² für H, Cl, Br, I oder CN steht und
Hal für Cl, Br oder I steht,
mit der Maßgabe, dass A, D und E nicht alle gleichzeitig dieselbe Bedeutung haben,
mit einem Fluorid in Gegenwart mindestens einer Verbindung der allgemeinen Formel (III), 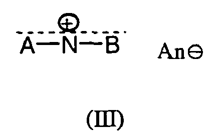 in der
A für einen Rest der Formeln (IV) oder (V) 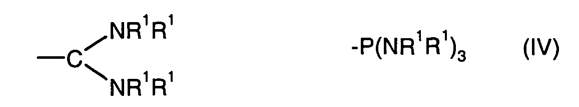
und
B unabhängig von A für einen Rest der Formeln (IV), (V), (VIa) oder (VIb) 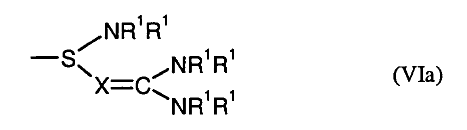
-S(NR¹R¹)₂ (VIb)
stehen,
wobei
die einzelnen R¹ gleich oder verschieden sind und jeweils für geradkettiges oder verzweigtes C₁-C₁₀-Alkyl, geradkettiges oder verzweigtes C₂-C₁₀-Alkylen oder C₆-C₁₂-Aryl stehen,
wobei eine oder mehrere NR¹R¹-Gruppen auch für einen 3- bis 5-gliedrigen, gesättigten oder ungesättigten Ring stehen können, der aus einem N-Atom und sonst C-Atomen gebildet wird,
wobei die Formel (IV) und die 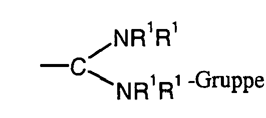 in Formel (VIa) auch für den Rest eines gesättigten oder ungesättigten 4- bis 8-gliedrigen Rings stehen kann, der zwei N-Atome und sonst C-Atome enthält,
X für N oder P steht und
An^{⊖} ein Äquivalent eines Anions bedeutet,
**dadurch gekennzeichnet, dass** die Umsetzung einstufig erfolgt.

2. Verfahren nach Anspruch 1, **dadurch gekennzeichnet, dass**
A und D für N stehen und E für CH, C-CF₃ oder C-CN steht oder
A und D für CH stehen und E für N, C-CF₃ oder C-CN steht oder
A für N steht, D für CH steht und E für C-CF₃ steht oder
A und D für CH stehen und E in der allgemeinen Formel (I) für CF und in der allgemeinen Formel (II) für C-Hal steht, wobei Hal die in Anspruch 1 genannte Bedeutung hat.

3. Verfahren nach Anspruch 1 oder 2, **dadurch gekennzeichnet, dass** die Verbindung der allgemeinen Formel (I) 4,5,6-Trifluorpyrimidin, 1,3,5-Trifluorbenzol oder 3,4,5-Trifluorbenzotrifluorid ist.

4. Verfahren nach wenigstens einem der Ansprüche 1 bis 3, **dadurch gekennzeichnet, dass** die Umsetzung in Gegenwart von Verbindungen der allgemeinen Formel (III) durchgeführt wird, worin die Reste R¹ für Methyl, Ethyl, Propyl oder Butyl oder eine NR¹R¹-Gruppe für einen 5- bis 7-gliedrigen gesättigten oder ungesättigten Ring, der aus einem Stickstoffatom und sonst C-Atomen gebildet wird
oder die Formel (IV) oder die 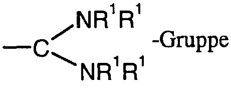 in Formel (VIa) für einen gesättigten 5- bis 7-
gliedrigen Ring, der 2 Stickstoffatome und sonst Kohlenstoffatome enthält,
X für Stickstoff und
An^{⊖} für Chlorid, Bromid, (CH₃)₃SiF₂^{⊖}, HF₂^{⊖}, H₂F₂^{⊖}, Tetrafluoroborat, Hexafluorophosphat, Carbonat oder Sulfat stehen.

5. Verfahren nach wenigstens einem der Ansprüche 1 bis 4, **dadurch gekennzeichnet, dass** wenigstens eine der Verbindungen der allgemeinen Formel (III) eine Verbindung der Formeln (III-1) bis (III-6) 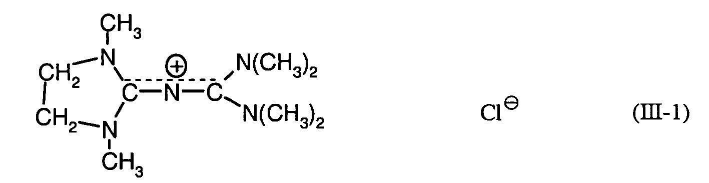 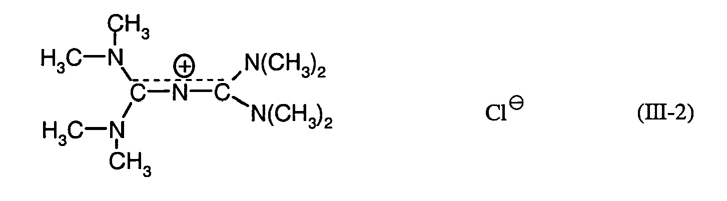 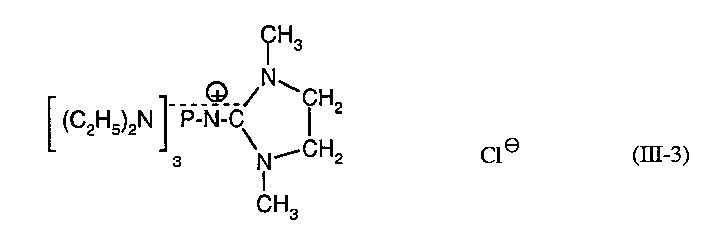 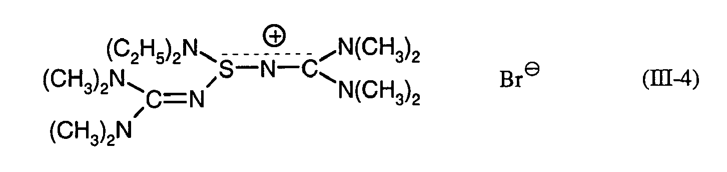 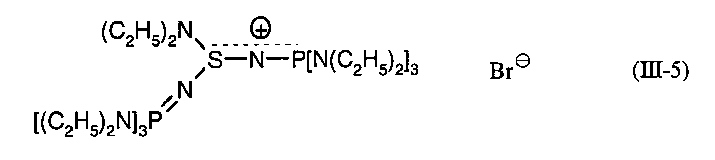 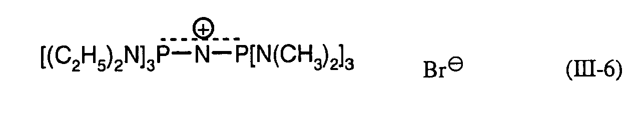 ist.

6. Verfahren nach wenigstens einem der Ansprüche 1 bis 5, **dadurch gekennzeichnet, dass** die Umsetzung bei wenigstens einer Temperatur von 40 bis 260°C durchgeführt wird.

7. Verfahren nach wenigstens einem der Ansprüche 1 bis 6, **dadurch gekennzeichnet, dass** bezogen auf 1 Mol gegen Fluor auszutauschendes Halogen, das an den Kern der Verbindung der allgemeinen Formel (II) gebunden ist, 0,001 bis 0,5 Mol einer oder mehrerer Verbindungen der Formel (III) und 0,8 bis 2 Äquivalente eines oder mehrerer Fluoride eingesetzt werden.

8. Verfahren nach wenigstens einem der Ansprüche 1 bis 7, **dadurch gekennzeichnet, dass** es in Gegenwart dipolar aprotischer und/oder unpolar aprotischer Lösungsmittel durchgeführt wird.

9. Verfahren nach wenigstens einem der Ansprüche 1 bis 8, **dadurch gekennzeichnet, dass** als Fluorid wenigstens ein Alkali-, Erdalkali- oder Ammoniumfluorid verwendet wird.

10. Verbindung der allgemeinen Formel (I), 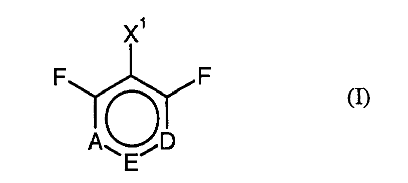
worin A, D, E und X¹, die in Anspruch 1 genannte Bedeutung haben, hergestellt nach dem Verfahren gemäß wenigstens einem der Ansprüche 1 bis 9.

11. Verwendung der Verbindung gemäß Anspruch 10 als Zwischenprodukte zur Herstellung von biologisch aktiven Substanzen für pharmazeutische und agrochemische Anwendungen.
